(19) Europäisches Patentamt

European Patent Office

Office européen des brevets

(11) Publication number: **0 312 700**
**A2**

## (12) EUROPEAN PATENT APPLICATION

(21) Application number: 88110556.3

(51) Int. Cl.4: **A61K 7/48** , **A61K 7/06**

(22) Date of filing: **01.07.88**

(30) Priority: **23.10.87 IT 2239787**

(43) Date of publication of application:
**26.04.89 Bulletin 89/17**

(84) Designated Contracting States:
**AT BE CH DE FR GB IT LI LU NL SE**

(71) Applicant: **Curti, Maria**
**Cascina San Leone**
**I-20070 Villanova Sillaro Milano(IT)**

(72) Inventor: **Curti, Maria**
**Cascina San Leone**
**I-20070 Villanova Sillaro Milano(IT)**

(74) Representative: **Bianchetti, Giuseppe**
**Studio Consulenza Brevettuale Via Rossini,**
**8**
**I-20122 Milan(IT)**

(54) **Cosmetic compositions.**

(57) Cosmetic compositions having anti-wrinkle, firming, anti-relaxant, anti-cellulitis, smoothing, distensive, anti-irritating, toning, etc. activities, containing from 0.2 to 5% of 3-N-(2-carboxyethyl)-aminobutyrric acid (CEGABA) or salts or esters thereof.

EP 0 312 700 A2

## COSMETIC COMPOSITIONS

The present invention refers to compositions having cosmetic activity (anti-wrinkle, firming, anti-cellulitis activities),containing, alone or in combination with other active principles, compounds of formula I:

$$XOOC-(CH_2)_2-NH-(CH_2)_3-COOY \quad (I)$$

wherein X and Y, which can be the same or different, are hydrogen, a $C_1-C_4$ alkyl residue or the cation of an alkali or alkali-earth metal.

The use of compounds of formula I, in particular of 3-N-(2-carboxyethyl)-aminobutyrric acid, or CEGABA (I, wherein X and Y = H) is well known in pharmaceutical compositions having a GABA-like activity at cerebral level; see EP-A-82200466.9, and also F. Savoldi et al., "Pharmacological Effects of CEGABA, a new amino acid occurring in mammalian brain", Il Farmaco, ed. sci.,52, 77-79 1987).

Also well known are compositions able to stimulate plants growth, containing as the active principle CEGABA itself and similar compounds (EP-0065678). Finally, Italian Patent 1 166 920 claims compositions for the topic treatment of scalp, able to stimulate the growth of new hair.

Now it has surprisingly been found that compounds of formula I have a remarkable cosmetic activity; in particular, they have surprisingly positive effects on the distension and smoothness of skin (anti-wrinkle effect); on its brightness, on the firmness and turgidity of tissues; on the decrease of cellulitis.

Compounds of formula I, and especially CEGABA, have equally favourable effects on after shave irritations, irritations in children caused by humid diapers or perspiration, feet irritation caused by maceration, irritations caused by sun exposure, and the like.

These activities are also present in cosmetic compositions per se known, to the active components of which one or more compounds of formula I have been added: in this case, an evident synergism between the latter and the already known active principles (e.g., collagen, hyaluronic acid, elastine and the like) is shown.

Therefore, object of the present invention are cosmetic compositions having anti-wrinkle, firming, anti-relaxant, anti-cellulitis, smoothing, distensive, anti-irritating, toning, hydrating, decongestive, anti-senescence, make-up removing, protecting, sebum-normalizing activities,or anyhow meant to improve the subjective and aesthetic look of the person, characterized in that they contain compounds of formula I, in particular CEGABA, alone or preferably in combination with principles having a similar activity. The content in compounds I, particularly of CEGABA, in the cosmetic formulations, may vary from 0.2 to 5% by weight, preferably from 0.5 to 1.5% by weight, and in most cases no particular advantage is observed when concentrations of compounds I, particularly of CEGABA, higher than 1.5% by weight, are used.

"Cosmetic compositions" of the present invention are creams, milks, lotions, ointments, oils, vials, masks, gels, buffers, sprays, for day and night use, for normal, dry or oily skins, for face and body, having a rapid absorption or to be applied with more or less prolonged massages, and to be used directly by the subject or by beauticians.

The activity of the cosmetic compositions according to the invention is shown by the following non-limiting examples. The percentages of the compositions are by weight.

## EXAMPLE 1

Commercial vials of 1% native collagen in water (vials A) for anti-wrinkle treatment, and identical vials containing more than 1% of CEGABA (vials B) were delivered to a beauty parlour. A weekly treatment for 4 weeks on two groups of 5 clients with vials A, and a weekly treatment for 4 weeks also on two groups of 5 clients with vials B were carried out.

The treatment with vials B gave, from the very first week, results remarkably higher than usual (distention and smoothness of the skin), according to the unanimous opinion both of the ten clients and of the beauticians.

## EXAMPLE 2

Some beauticians received a creamy emulsion available on the market for anti-wrinkle treatment, having a 1% collagen content (emulsion A), and the same emulsion added with 1% of CEGABA. Daily domicile treatments were carried out on ten clients per product, for two weeks. All the twenty clients had been using the above emulsion for a long time.

The subjective results were the following:
- the ten clients treated with emulsion A obviously did not notice any difference with respect to the previous treatments;
- all the ten clients treated with emulsion B declared that, from the second day of treatment, the result was remarkably improved as far as distention and smoothness of the skin were concerned.

## EXAMPLE 3

Two beauticians received:
- a firming aqueous solution, already available on the market, containing 5% of elastine and 1% of hyaluronic acid (solution A);
- the same solution, added with 1% of CEGABA (solution B);
- a firming creamy emulsion, already available on the market, containing 0.5% of elastine and 0.5% of hyaluronic acid (emulsion A);
- the same emulsion, added with 1% of CEGABA (emulsion B).

With the first two formulations (solutions A and B), six clients received breast massages, and six neck massages, twice a week for four weeks. The twelve cases treated with solution A, only after the sixth application, showed really visible results, in full agreement with the ones normally obtained with said solution; on the contrary, already after the fourth application, all the twelve cases treated with solution B showed remarkably higher results in terms of both firmness and smoothness.

The tests with emulsions A and B were carried out at domicile, each on eight clients (four for breast and four for neck), who had been using for a long time the commercial firming product herein called emulsion A.

The cases treated with said emulsion did not show, obviously, any difference with respect to the previous treatments; emulsion B, on the contrary, gave on all the eight clients results that were considered "definitely better" both in the rapidity of action and in the extent of the effect (firmness and elasticity).

## EXAMPLE 4

Ten cases with marked cellulitis problems in the thighs were treated twice a week for five weeks with a half an hour massage:
- with an anti-cellulitis product available on the market (vials A), containing 2.5% of lipolytic enzymes, on one thigh;
- with the same product, added with 1% of CEGABA (vials B), on the other thigh.

The decrease in the diameter of the thigh treated with vials A (average of the ten cases) at the end of the treatment was of 2.5 cm; for the thigh treated with vials B, also as an average of the ten cases, and at the end of the treatment, the decrease was of 4 cm.

Equally interesting results were also obtained by adding CEGABA, or derivatives thereof of formula I, to cosmetic products having a lenitive action on local irritation (after shave creams and lotions, creams to treat juvenile acne, creams and lotions for manicure, pedicure, etc.).

## EXAMPLES OF FORMULATIONS

a) Hydroglycolic tonic solution containing Hamamelis natural distilled water; lime-tree, rose and Hamamelis extracts; moisturing factor; native collagen; elastine; hyaluronic acid; CEGABA 0.6-1.2%.

b) Day cream for normal skins (anti-wrinkle, firming and hydrating): creamy emulsion containing corn germ oil; olive oil, hyaluronic acid; elastine; collagen; CEGABA 0.8-1.2%.

c) Day cream for dry skins (anti-wrinkle, firming and hydrating): creamy emulsion containing avocado, corn germ, carrot and silicone oils; karitè butter; dogrose extract; collagen; elastine; mucopolysaccharides; CEGABA 0.8-1.2% or the diethyl ester thereof; emulsifying agents and preservatives according to EEC regulations.

d) Rich cream for very dry skins with tendency to desquamation (anti-wrinkle, firming and hydrating): thick creamy emulsion containing isopropyl myristate; vaseline oil; proteic hydrolized products from collagen and elastine; mucopolysaccharides; CEGABA 0.8-1.2%.

e) Special night cream: thick creamy emulsion containing dogrose extract; carrot and corn germ oil; karitè butter; silicone oil; collagen; elastine; hyaluronic acid; CEGABA 0.8-1.2% or the diethyl ester thereof.

f) Anti-wrinkle vials: thick liquid, containing collagen; elastine; silicone oil; CEGABA 0.8-1.2%.

g) Firming and toning vials: thick liquid, containing collagen; elastine; hyaluronic acid; dermatansulfate; CEGABA 0.7-1.3% or the diisopropyl ester thereof.

h) After shave lotion cream: fluid milk containing corn germ and olive oil; hyaluronic acid; elastine; collagen and CEGABA 1%.

i) Body cream (smoothing, hydrating, distensive): fluid emulsion containing olive and corn germ oil; hyaluronic acid; elastine; collagen; CEGABA 0.8-1.2% or the diethyl ester thereof.

l) Quick absorption firming cream: creamy emulsion containing glucosamine hydrochloride; dogrose glycol extract; hyaluronic acid; native collagen; elastine; CEGABA 0.8-1.2%.

m) Firming cream for massages: creamy emulsion; the content is the same as in l).

n) Quick absorption slimming cream: creamy emulsion containing sea oak; horse-chestnut, ivy, hop, centella asiatica extracts; collagen; elastine; mucopolysaccharides; CEGABA about 1% or the diethyl ester thereof.

o) Slimming cream for massages: creamy emulsion; the content is the same as in n).

p) Anti-cellulitis vials (powder to be dissolved before use): they contain lipolytic enzymes + CEGABA 0.8-1.2%.

q) Sun cream, fluid emulsion: it contains isopropyl myristate; carrot oil; collagen hydrolized products; elastine; mucopolysaccharides; CEGABA about 1%.

r) Protective sun milk, fluid emulsion: it contains avocado, carrot, silicone and corn germ oils; karitè butter; dogrose extract; native collagen; 3,4-methylbenzylidenebornanone; CEGABA 0.8-1.2%.

s) Thick emulsion for manicure: it contains olive and corn germ oil; collagen; elastine; CEGABA about 1%.

t) Creamy emulsion for pedicure: it contains menthol crystals; mentha crispa essential oil; natural camphor; CEGABA 0.7-1.3%

All the above examples are illustrative in scope; the nature of the components, the amount, the consistency, the aspect of the various compositions may range within wide limits, the only characterizing aspect being the presence in them of CEGABA or derivatives thereof of formula I.

## Claims

1. Cosmetic compositions characterized in that they contain, alone or combined with other active principles, one or more compounds of formula I

$$XOOC\text{-}(CH_2)_2\text{-}NH\text{-}(CH_2)_3\text{-}COOY \qquad (I)$$

wherein X and Y, which can be the same or different, are hydrogen, straight or branched $C_1\text{-}C_4$ alkyl residues, or cations of alkali or alkali-earth metals.

2. Cosmetic compositions according to claim 1, characterized in that they contain, as compound of formula I, N-(2-carboxyethyl)-3-aminobutyrric acid, or CEGABA (formula I, wherein X = Y = H).

3. Cosmetic compositions according to claims 1 and 2, characterized in that they contain from 0.2 to 5% by weight of compounds of formula I.

4. Cosmetic compositions according to claim 3, characterized in that they contain from 0.5 to 1.5% by weight of the compounds of formula I.

5. Cosmetic compositions according to claims 1-4, having anti-wrinkle, firming, anti-relaxant, anti-cellulitis, smoothing, distensive, anti-irritating, toning, hydrating, decongestive, anti-senescence, anti-acne, detergent, make-up removing, protecting, sebum-normalizing activities, or anyhow meant to improve the subjective and aesthetic look of a person.

6. Cosmetic compositions according to claims 4-5, characterized in that they contain about 1% by weight of compounds of formula I.

7. Cosmetic compositions according to claims 1-6, in form of lotions, milks, creams, ointments, oils, vials, masks, gels, buffers, sprays.

8. Use of compounds of formula I:

$$XOOC\text{-}(CH_2)_2\text{-}NH\text{-}(CH_2)_3\text{-}COOY \qquad (I)$$

wherein X and Y, which can be the same or different, are hydrogen, straight or branched $C_1\text{-}C_4$ alkyl residues, or cations of alkali or alkali-earth metals,

for the preparation of cosmetic compositions for cutaneous administration.